Europäisches Patentamt

European Patent Office

Office européen des brevets

(19)

(11) Numéro de publication : **0 459 842 A1**

(12)

# DEMANDE DE BREVET EUROPEEN

(21) Numéro de dépôt : **91400991.5**

(22) Date de dépôt : **15.04.91**

(51) Int. Cl.⁵ : **A61K 39/21, C07K 3/12**

(30) Priorité : 09.05.90 FR 9005775

(43) Date de publication de la demande :
04.12.91 Bulletin 91/49

(84) Etats contractants désignés :
AT BE CH DE FR GB IT LI NL SE

(71) Demandeur : PASTEUR MERIEUX SERUMS ET
VACCINS, Sociéte Anonyme :
58 Avenue Leclerc
F-69348 Lyon Cédex 07 (FR)

(72) Inventeur : Pelloquin, François
30 avenue des Frères Lumière
F-69008 Lyon (FR)
Inventeur : Heimendinger, Pierre
52 Boulevard Jean XXIII
F-69008 Lyon (FR)

(74) Mandataire : Bernasconi, Jean et al
CABINET LEMOINE ET BERNASCONI 13,
Boulevard des Batignolles
F-75008 Paris (FR)

(54) **Procédé de fabrication d'immunogènes de rétrovirus et de vaccins contre les infections rétrovirales, notamment HIV, et immunogènes et vaccins obtenus.**

(57)    Le surnageant d'une culture de particules rétrovirales tel que HIV est inactivé à la bétapropiolactone en deux étapes à température basse et à température élevée, concentré par ultrafiltration, soumis à une gamma irradiation, purifiée, de préférence, par dialyse, gel filtration et chromatographie d'échange d'ions, puis chauffé à 60°C pendant au moins une heure. Application aux immunogènes et vaccins HIV.

EP 0 459 842 A1

EP 0 459 842 A1

La présente invention a trait à un procédé de fabrication d'immunogènes de rétrovirus et de vaccins contre les infections rétrovirales ainsi qu'aux immunogènes et aux vaccins obtenus. L'invention s'applique notamment aux virus de l'immunodéficience humaine (HIV) mais peut également s'appliquer à d'autres rétrovirus humains, notamment HTLV-1 et HTLV-2, et animaux, par exemple BLV.

L'extraordinaire effort de recherche qui a été provoqué par l'apparition et le développement, sur le mode d'une pandémie, des infections à HIV, a abouti à l'isolation ou la construction de nombreux antigènes dérivant de HIV et d'autres rétrovirus. Cependant, si ces résultats ont fait faire des progrès importants dans le domaine de la détection des infections rétrovirales, et notamment HIV, la recherche d'un vaccin protecteur a abouti à de nombreuses déceptions.

Une nouvelle approche du problème a été proposée dans la demande de brevet PCT WO 88/09670 Jonas SALK et Dennis J. CARLO qui a abouti à la réalisation d'un immunogène constitué de virions non infectieux, en fait inactivés, et dépourvus de glycoprotéines d'enveloppe. Dans le cas du virus HIV, ces immunogènes, qui seront appelés ci-après de type SALK, sont dépourvus de glycoprotéines d'enveloppe gp160 ou gp120. Il est renvoyé à cette demande PCT, ainsi qu'aux autres publications intervenues, pour l'énoncé des considérations théoriques à la base de cette approche et qui peuvent se résumer, en première approximation, à l'intérêt d'éviter l'induction d'anticorps anti-enveloppe dont le rôle facilitant l'infection est maintenant reconnu et à la recherche d'une protection à médiation cellulaire.

La demande de brevet précitée propose également un procédé de fabrication de ces immunogènes et vaccins HIV (qui englobent non seulement HIV-1 mais également HIV-2) par culture de cellules infectées par HIV, dans un milieu convenable, en l'occurrence le milieu RPMI 1640 en présence de 10 % de sérum de veau foetal en tampon 25 mM HEPES, additionné d'antibiotiques. Après filtration du surnageant, il est procédé à une inactivation à la béta-propiolactone pendant 5 heures à pH sensiblement neutre à 37°C à une concentration au 1:4000. Le surnageant congelé est ensuite soumis à une gamma irradiation puis, après décongélation, concentré par ultrafiltration, puis purifié par une succession d'ultracentrifugations, l'étape de gamma irradiation pouvant d'ailleurs également suivre les étapes de purification.

Ce procédé, au demeurant contrôlé par des vérifications poussées, permet d'obtenir, à partir d'un litre de surnageant, une dizaine de doses de type SALK comprenant environ 100 µg/ml de protéines totales.

Ce procédé, qui doit être mis en oeuvre par des techniciens hautement qualifiés, ne se prête pas à une exploitation industrielle, non seulement à cause de son faible rendement, mais encore à cause des risques qui peuvent être liés à une disparition incomplète des propriétés infectieuses, notamment dans le cas du traitement de très grands volumes, sans parler de la nécessité d'obtenir, dans le but de présenter un vaccin pouvant être administré systématiquement, et notamment un vaccin de prévention, une pureté extrêmement poussée.

Il est d'ailleurs à remarquer que, compte tenu du principe même des immunogènes de type SALK, il est extrêmement difficile et même apparemment contradictoire de vouloir augmenter à la fois le rendement, la pureté, la certitude de l'absence d'infectivité et le pouvoir immunogène et protecteur.

La présente invention se propose de fournir un tel procédé de fabrication d'immunogènes de rétrovirus et de vaccins contre les infections rétrovirales, notamment HIV, pouvant être mis en oeuvre de façon industrielle dans des conditions de sécurité optimales pour le personnel, en obtenant un rendement très important, un degré de pureté extrêmement poussé et une certitude d'absence de caractère infectieux, et ceci sans perturber les propriétés immunogènes et protectrices de la préparation particulaire antigénique obtenue.

L'invention a pour objet un procédé de fabrication d'immunogènes de rétrovirus et vaccins contre les infections rétrovirales, notamment HIV, de type SALK, dans lequel on produit, par culture de cellules infectées, une récolte, de préférence dans un surnageant, de particules rétrovirales comprenant leur enveloppe, on ajoute à la suspension de particules, au besoin préalablement filtrée, un agent inactivant viral usuel dégradant l'enveloppe, tel que la béta-propiolactone par exemple, on concentre la suspension, on la soumet à une gamma-irradiation et on la purifie, caractérisé en ce qu'aux étapes de traitement de l'enveloppe par ledit agent et d'irradiation, on associe une étape de chauffage de longue durée et une étape de purification permettant l'élimination des glycoprotéines extérieures d'enveloppe.

Conformément à une caractéristique préférée de l'invention, on prévoit une étape de traitement à l'aide d'un agent dégradant l'enveloppe virale, de préférence la béta-propiolactone, comprenant une première étape d'incubation, de longue durée à faible température, suivie d'une deuxième étape d'élévation de température. La première étape peut permettre à l'agent, tel que la béta-propiolactone, de dissocier l'enveloppe rétrovirale et d'agréger les protéines extérieures d'enveloppe, telles que gp120 ou gp160 pour HIV, pendant une durée importante, de préférence supérieure ou égale à 16 heures à une température basse, de préférence de 4 à 8°C, alors que la deuxième étape de chauffage, de préférence à une température de l'ordre de 37±1°C, pendant une durée d'au moins 3 heures, assure une dégradation de l'agent inactivant tout en préservant les éléments antigéniques désirables des particules virales.

Conformément à une caractéristique préférée de l'invention, la gamma-irradiation est effectuée à une dose

2

de l'ordre de 45 kGray.

Conformément à une caractéristique également préférée de l'invention, l'étape de chauffage est effectuée en milieu liquide pendant une durée d'au moins une heure à une température de l'ordre de 60°C et l'on peut parvenir à l'inactivation totale de la transcriptase inverse rétrovirale tout en conservant les propriétés antigéniques recherchées.

Les étapes de purification proprement dites sont destinées, comme dans le procédé antérieurement décrit, à éliminer les protéines étrangères, et notamment les protéines en provenance du milieu de culture. Cependant, conformément à une caractéristique préférée de l'invention, ces étapes comprennent une étape d'élimination des glycoprotéines rétrovirales extérieures d'enveloppe, effectuée dans des conditions de pH très acide et l'on préfère, à cette fin, une étape de diafiltration à pH de l'ordre de 4,50 ou 4,70, par exemple en acétate de sodium 100 mM en présence de NaCl 2M, cette étape étant de préférence suivie par une étape de diafiltration destinée au rééquilibrage du pH et de la force ionique.

L'ordre de succession des quatre étapes de traitement à la béta-propiolactone ou d'un autre agent, de préférence, classique, de dégradation d'enveloppe, par exemple le formaldéhyde, de gamma-irradiation, de chauffage et de purification éliminant les glycoprotéines extérieures d'enveloppe, peut être quelconque.

Cependant, on préférera que l'étape de traitement par la béta-propiolactone ou autre agent précède l'étape de purification de façon à mettre à profit la formation d'agrégats de protéines externes plus faciles à éliminer.

L'étape de gamma-irradiation sera effectuée à un moment quelconque du cycle, en fonction, en fait, des nécessités techniques.

Quant à l'étape de chauffage, on préférera la mettre en oeuvre dans une phase finale du procédé afin de faire porter le chauffage sur un produit déjà hautement purifié et dépourvu de protéines exogènes et, de préférence, dans les récipients utilisés dans l'installation dans laquelle on effectue l'ajustement de la concentration en protéines virales.

Outre la dégradation irréversible de la transcriptase inverse, l'étape de chauffage provoque encore, s'il en est besoin, la rupture de la capside et la dégradation de l'enveloppe et de ses constituants glycoprotéiques.

De préférence, on procède, à plusieurs étapes du procédé selon l'invention, à des congélations suivies de décongélations destinées à provoquer la rupture des formations protéiques de l'enveloppe et de la capside rétrovirales, les étapes de congélations pouvant en outre être utilisées pour le transport de la préparation en cours de traitement entre différentes installations, et notamment vers l'installation de gamma-irradiation, en général extérieure à l'installation où s'effectue l'essentiel des étapes de fabrication.

Comme cela a été précisé, les étapes de purification, qui peuvent avantageusement mettre à profit des étapes précédentes, notamment de traitement à la béta-propiolactone et de concentration permettant de préférence l'élimination d'une quantité importante des protéines étrangères, par exemple les protéines bovines, comportent un ensemble d'étapes de diafiltration assurant l'élimination des protéines rétrovirales externes d'enveloppe. Ces étapes de diafiltration comprennent, de préférence, un premier stade de diafiltration permettant l'élimination des protéines bovines, par exemple à pH 7,25, puis le stade d'élimination des glycoprotéines d'enveloppe, de préférence en tampon acide à pH très acide, et enfin un troisième stade de rééquilibrage de la force ionique et du pH.

Cependant, dans d'autres modes de mise en oeuvre, moins préférés, de l'invention, une élimination de glycoprotéines extérieures d'enveloppe peut être obtenue par dissociation par des détergents anioniques, tels que Triton, SDS, Tween, NP40 ou des solvants organiques du type ether/chloroforme, suivie d'une séparation.

De façon avantageuse, on peut ensuite procéder à une gel-filtration permettant l'élimination de l'albumine bovine restante, suivie, si nécessaire, d'un passage sur échangeur d'ions permettant l'élimination des protéines résiduelles.

Une ultracentrifugation peut ensuite être mise à profit pour éliminer le tampon utilisé lors de la récupération des éléments d'origine virale sur la colonne échangeuse et provoquer une nouvelle concentration, le culot étant ensuite repris en milieu liquide stérile, ajusté si besoin à la concentration désirable, puis soumis, de préférence à ce stade, à l'étape de chauffage. On obtient à ce niveau un produit dit produit final vrac PFV constitué d'une suspension d'antigènes purifiés, avec un rendement de plus de 50 doses par litre de surnageant initial.

Le procédé selon l'invention peut être complété par des opérations d'élimination des débris d'acides nucléiques, par exemple par ultracentrifugations successives.

Le produit final vrac peut alors être conditionné soit sous forme de doses vaccinales après addition d'un adjuvant, soit sous forme de doses de test d'intradermoréaction (IDR) aux dilutions appropriées.

Le procédé selon l'invention peut partir d'un surnageant de culture de rétrovirus sur des cellules convenablement infectées. Les modalités et conditions de cultures ne sont pas déterminantes.

Dans le cas de HIV, il est intéressant d'utiliser une lignée cellulaire infectée mais viable ayant intégré le provirus rétroviral.

A titre d'exemple, on peut utiliser une lignée CEM dérivant de lymphocytes T humains infectés par le virus

EP 0 459 842 A1

HIV LAV, disponible chez le Professeur Montagnier à l'Institut Pasteur. La lignée CEM a fait l'objet d'un dépôt ATCC n° CCL 119.

Cette lignée peut également être infectée par d'autres souches de virus HIV-1. D'autres lignées cellulaires chroniquement infectées par HIV peuvent être utilisées, par exemple la lignée HB2 ou A 102.

L'invention a également pour objet les immunogènes de rétrovirus, y compris de rétrovirus HIV-1 ou HIV-2, de HTLV I, HTLV II ou d'autres rétrovirus humains ou animaux, obtenus par le procédé selon l'invention et se caractérisant par leur immunogénicité.

Ces immunogènes peuvent être utilisés, par exemple, pour la détermination par intradermoréaction de la réponse immunitaire de l'organisme. Les différentes dilutions pour l'IDR peuvent être par exemple de 0,1, 1 et 10 µg par ml de protéines totales dont l'essentiel est d'origine virale.

L'invention a également pour objet les vaccins obtenus par le procédé selon l'invention, notamment des vaccins anti-HIV, et ces vaccins étant de préférence adjuvés à l'aide d'adjuvants classiques tels que par exemple adjuvant incomplet de Freund et conditionnés par exemple sous dose de 1 ml adjuvé avec une teneur de préférence de l'ordre de 100 µg/ml de protéines totales.

Dans le cas du virus HIV, on peut, si on le souhaite, mélanger les immunogènes provenant de souches différentes de virus HIV.

D'autres avantages et caractéristiques de l'invention apparaîtront à la lecture de la description suivante, faite à titre d'exemple non limitatif relatif à la préparation d'immunogènes et de vaccins de type SALK à partir de HIV-1.

### 1° - Lignée cellulaire infectée.

La lignée cellulaire utilisée est une lignée cellulaire appelée CEM/HIV-1. Cette lignée est obtenue à partir de la lignée CEM, dérivée de lymphocytes T humains, n° ATCC CCL 119 infectée par le virus LAV1 fourni le Professeur Montagnier.

Pour obtenir la lignée CEM/HIV-1, on infecte un certain nombre de cellules de CEM avec des concentrations différentes de LAV1 et l'on constate, de façon reproductible, que la culture infectée avec une faible concentration de virus (virus dilué $1/10^5$) permet d'obtenir, après criblage, un ou des clones viables, c'est-à-dire ayant intégré le provirus LAV dans leur génomes.

Le stock CEM/HIV-1 conservé en azote liquide forme la banque cellulaire de travail.

### 2° - Culture.

Un certain nombre de tubes de la banque cellulaire de travail sont décongelés et les cellules mises en culture en suspension dans un milieu RPMI-1640 en présence de 5 % de sérum de veau foetal et de 2 g/l de bicarbonate et en l'absence d'antibiotique. Après amplification, on ensemence un biogénérateur de 10 l à raison de $5.10^9$ cellules CEM/HIV-1, la densité initiale étant donc de 0,5 $10^6$/ml.

La culture est effectuée à 37°C dans le même milieu et l'on constate qu'à trois à quatre jours, la concentration cellulaire est de 1 à 1,5 $10^6$/ml. A ce moment, on procède à un soutirage d'environ 8 litres et l'on remplace le surnageant ainsi soutiré par un milieu de culture neuf et l'on continue le processus un certain nombre de fois.

Les surnageants soutirés sont mélangés en pool.

### 3° - Préparation du produit final vrac (immunogène purifié)

On se réfère au tableau I.

### a) filtration

100 l de surnageant de culture sont soumis à deux filtrations consécutives à 5 µm puis 0,45 µm, formant 100 l de récolte brute. Activité infectieuse $10^6$ doses infectieuses/ml. Activité transcriptase inverse $10^6$ cpm/ml.

### b) inactivation

Les 100 l de surnageant sont inactivés par la bétapropiolactone à 4°C pendant 16 heures à une concentration en béta PL au 1/4000. Après 16 heures, la suspension est amenée à 37°C et maintenue à cette température pendant au moins trois heures.

Activité infectieuse non détectable. Activité RT $10^5$ cpm/ml.

4

c) ultrafiltration.

Les 100 l de suspension inactivés sont soumis à une ultrafiltration tangentielle à une température comprise entre 4 et 10°C sur un ultrafiltre 300.000 daltons. Cette étape élimine la plupart des protéines bovines et aboutit à un ultrafiltrat qui a été concentré 50 fois. L'ultrafiltrat est congelé à - 70°C.

d) gamma irradiation.

Les deux litres d'ultrafiltrat congelés sont amenés à l'installation d'irradiation et soumis à l'irradiation aux rayons gamma de 45 kGray. L'ultrafiltrat toujours congelé, est ramené et décongelé.
Activité infectieuse. non détectable Activité RT $5.10^3$ cpm/ml

e) diafiltration

Les deux litres, décongelés à une température entre + 4°C et 10°C, sont diafiltrés (seuil de coupure : 300.000 daltons) successivement en trois tampons :
1) Tris HCl 25 mM, pH = 7,25, NaCl 10 mM.
2) La diafiltration est ensuite poursuivie en milieu acétate Na 100 mM, pH = 4,65, NaCl 2 M.
3) La diafiltration est ensuite poursuivie en milieu acétate Na 50 mM pH = 6,5. On obtient un litre de suspension concentrée deux fois, dépourvue de la plus grande part des produits d'origine bovine et dont les glycoprotéines externes d'enveloppe de type gp120, gp160 sont pratiquement totalement éliminées.
Activité infectieuse indétectable. Activité $RT 5.10^3$ cpm/ml.
Un Western Blot confirme l'élimination des gp 120, 160.

f) poursuite de la purification

La suspension provenant de la diafiltration, d'un volume de 1 l, est soumise à une gel-filtration sur Sephacryl S500HR (Pharmacia) en tampon acétate de 100 mM pH = 6,6. Cette étape poursuit l'élimination de l'albumine bovine et aboutit à un volume de 5 l de suspension purifiée.
La suspension de 5 l obtenue est ensuite soumise à un passage sur échangeur d'ions CM Sepharose Fast Flow. On effectue le premier passage en acétate de Na 100 mM pH = 6,5 pour la fixation des antigènes et l'élution des protéines résiduelles. On procède ensuite à un rinçage de la colonne en tampon phosphate de Na 10 mM, pH = 6,5 et l'on procède enfin à l'élution des antigènes en tampon phosphate de Na 200 mM, pH = 7,5. La suspension formée par l'éluat présente un volume d'environ 1 l.
Cette suspension est soumise à une ultracentrifugation en coussin de saccharose (sucrose) à 30 % à 28000 tr/min. pendant 1 heure à + 4°C sur rotor SW28 ou TI.45. Le culot, contenant les antigènes recherchés, est ensuite repris en soluté apyrogène NaCl 9/1000 stérile, puis ajusté à 200 µg/ml de protéines totales correspondant à un volume de suspension d'environ 2,5 l. La suspension est légèrement opalescente.

g) chauffage.

La suspension ajustée est chauffée une heure à 60°C.
Activité infectieuse indécelable-Activité RT indécelable.
La suspension chauffée qui forme le produit final vrac peut être congelée.

4° - Utilisation du Produit Final Vrac

Le produit final vrac (2,5 l à 200 µg/ml de protéines totales) est mélangé volume pour volume, à un adjuvant incomplet de Freund. Le mélange est ensuite réparti en doses unitaires de 1 ml adjuvé contenant 100 µg de protéines virales antigéniques formant la dose vaccinale.
Pour faire un produit de test cutané IDR, on utilise le produit final vrac non adjuvé, conditionné en dilutions de 0,1, 1, et 10 µg/ml de protéines totales.

Soutirage

Filtrations $\qquad$ $5\mu$ ; $0,45\mu$

100 l

Inactivation $\beta$PL 1/4000 $\qquad$ $\geqslant$ 16 h 4-8°C puis
$\geqslant$ 3 h 37 $\pm$1°C

100 l

Ultrafiltration 300000 4°C

2 l

Congélation

$\gamma$ irradiation

décongélation

2 l

1) Tris HCl 25 mM pH = 7,25
NaCl 10 mM

Diafiltration 300000 $\qquad$ 2) Acétate de Na 100 mM
NaCl 2 M pH = 4,65

1 l $\qquad$ 3) Acétate de Na 50 mM
pH = 6,5

Gel filtration   Acétate de Na 100 mM
pH = 6,5

5 l

1) Acétate de  Na
100 mM pH 6,5

Echange   2) Phosphate Na 10  mM
d'ions        pH = 6,5

3) Phosphate Na 200 mM
pH = 7,5

1 l

ultracentrifugation   28000 RPM Saccharose 30 %
1h SW 28 + 4°C

culot

reprise du culot et ajustement   NaCl 9°/oo

2,5 l

Chauffage $\qquad$ $\geqslant$ 1 h 60°C

PFV

**Revendications**

1. Procédé de fabrication d'immunogènes de rétrovirus ou de vaccins contre les infections rétrovirales, notamment HIV, dans lequel on produit, par culture de cellules infectées, une récolte de particules rétrovirales comprenant leur enveloppe, en supension, on ajoute à la suspension de particules, un agent inactivant viral usuel dégradant l'enveloppe, on concentre la suspension, on la soumet à une gamma irradiation et on la purifie, caractérisé en ce qu'aux étapes de traitement de l'enveloppe par ledit agent et d'irradiation, on associe une étape de chauffage de longue durée et une étape de purification permettant l'élimination des glycoprotéines extérieures d'enveloppe.

2. Procédé selon la revendication 1, caractérisé en ce que l'étape de traitement d'inactivation s'effectue à l'aide de la bétapropiolactone, avec une première étape d'incubation de longue durée à faible température suivie d'une deuxième étape d'élévation de température permettant d'inactiver l'agent.

3. Procédé selon la revendication 2, caractérisé en ce que l'on effectue une inactivation à une température de l'ordre de 4 à 8°C pendant une durée supérieure ou égale à 16 h suivie d'une élévation de température de l'ordre de 37°C pendant une durée d'au moins trois heures.

4. Procédé selon l'une quelconque des revendications 1 à 3, caractérisé en ce qu'on effectue l'étape de gamma-irradiation à une dose de l'ordre de 45 kGray.

5. Procédé selon l'une quelconque des revendications 1 à 4, caractérisé en ce que l'étape de chauffage est effectuée en milieu liquide pendant une durée d'au moins une heure à une température de l'ordre de 60°C.

6. Procédé selon l'une quelconque des revendications 1 à 5, caractérisé en ce que l'on effectue l'étape de chauffage après les autres étapes.

7. Procédé selon l'une quelconque des revendications 1 à 6, caractérisé en ce qu'il comporte une étape de purification assurant l'élimination des glycoprotéines rétrovirales extérieures d'enveloppe dans des conditions de pH acide.

8. Procédé selon la revendication 7, caractérisé en ce que ladite étape d'élimination comprend une étape de diafiltration à un pH de 4,50 à 4,70.

9. Procédé selon la revendication 8, caractérisé en ce que l'on effectue ladite étape de dialyse en acétate de sodium 100 mM en présence de NaCl 2 M, pH = 4,65.

10. Procédé selon l'une quelconque des revendications 1 à 9, caractérisé en ce que l'étape d'inactivation précède l'étape de purification.

11. Procédé selon l'une quelconque des revendications 1 à 10, caractérisé en ce que l'on procède à des congélations suivies de décongélations provoquant la rupture des formations protéiques de l'enveloppe et de la capside rétrovirales.

12. Procédé selon l'une quelconque des revendications 1 à 11, caractérisé en ce qu'après une étape de diafiltration assurant l'élimination des protéines rétrovirales externes d'enveloppe, on procède à une gel filtration dans des conditions éliminant les protéines exogènes, suivie, si nécessaire, d'un passage sur échangeur d'ions.

13. Procédé selon l'une quelconque des revendications 1 à 12, caractérisé en ce que l'on élimine le tampon utilisé lors de l'échange d'ions par ultracentrifugation en milieu saccharose.

14. Procédé selon l'une quelconque des revendications 1 à 13, caractérisé en ce que la suspension de particules est concentrée par ultrafiltration après le traitement par l'agent inactivant.

15. Prépration d'immunogènes, notamment HIV, caractérisée en ce qu'elle est obtenue par le procédé selon l'une quelconque des revendications 1 à 14.

16. Préparation de test IDR, caractérisée en ce qu'elle comporte un immunogène selon la revendication 15.

17. Vaccin contre les infections rétrovirales, notamment HIV, caractérisé en ce qu'il contient une préparation selon la revendication 15.

18. Vaccin selon la revendication 17, caractérisé en ce qu'il comporte un adjuvant.

Office européen
des brevets

**RAPPORT DE RECHERCHE EUROPEENNE**

Numero de la demande

EP    91 40 0991

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,D | WO-A-8809670 (THE IMMUNE RESPONSE CORPORATION) * le document en entier * | 1, 4, 14-15, 17-18 | A61K 39/21 C07K 3/12 |
| A | WO-A-8705911 (HOFMANN,BO) * page 17; revendication 1 * | 1, 5 | |

DOMAINES TECHNIQUES
RECHERCHES (Int. Cl.5)

C12N
C12P
C07K

Le présent rapport a été établi pour toutes les revendications

| Lieu de la recherche | Date d'achèvement de la recherche | Examinateur |
|---|---|---|
| LA HAYE | 17 JUILLET 1991 | FERNANDEZ Y BRA F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)